# FASCICULE DE BREVET EUROPEEN

(11) **EP 1 090 622 B1**
(45) Date de publication et mention de la délivrance du brevet: **22.01.2003**
(21) Numéro de dépôt: 00402658.9
(22) Date de dépôt: 26.09.2000
(51) Int. Cl.: A61K 7/06

(54) **Composition de lavage des matières kératiniques, à base d'un agent tensio-actif détergent, d'une silicone fonctionnalisée et d'un terpolymère acrylique**
Haarshampoo auf Basis von ein Tensid, einer funktionalisierten Silicon und ein Acryl-Terpolymer
A washing composition for keratinous materials based on a surfactant, a functionalised silicone and an acrylic terpolymer

(30) Priorité: 29.09.1999 FR 9912170
(43) Date de publication de la demande: 11.04.2001
(73) Titulaire: L'OREAL, 75008 Paris (FR)
(72) Inventeur: Maurin, Véronique, 75016 Paris (FR); Beauquey, Bernard, 92110 Clichy (FR)
(74) Mandataire: Casalonga, Axel

(56) Documents cités:
- EP-A- 0 824 914
- EP-A- 0 825 200
- WO-A-97/35545
- FR-A- 2 779 639
- US-A- 5 573 709

## Description

La présente invention concerne d'une manière générale des compositions de lavage des matières kératiniques, à base d'un agent tensio-actif détergent, d'une silicone fonctionnalisée et d'un terpolymère acrylique, ainsi qu'un procédé de lavage mettant en oeuvre ces compositions.

Les silicones fonctionnalisées sont généralement utilisées dans les compositions de shampooings en tant qu'agents conditionneurs pour améliorer la douceur, le toucher et le démêlage des cheveux. Cependant, on a constaté que ces silicones conduisaient à la formation d'une couche inesthétique à la surface du shampooing. Pour éviter l'apparition de ce phénomène, des agents de stabilisation tels que les polymères acryliques réticulés du type Carbopol sont fréquemment utilisés. Néanmoins, ces agents de stabilisation présentent l'inconvénient de diminuer les performances cosmétiques des shampooings, notamment en rendant les cheveux plus chargés et plus rêches.

Il était donc nécessaire de mettre au point une composition cosmétique détergente, en particulier un shampooing, qui ait un aspect esthétique satisfaisant tout en conférant des performances cosmétiques acceptables aux matières kératiniques, à savoir notamment les cheveux et le cuir chevelu.

La demanderesse a découvert, de façon surprenante, qu'il était possible de formuler des compositions de lavage des matières kératiniques, notamment des shampooings, ayant les propriétés recherchées, en utilisant dans ces compositions un agent tensio-actif détergent et une silicone fonctionnalisée associés à un terpolymère acrylique spécifique, défini ci-après. En effet, il a été constaté que l'utilisation dudit terpolymère acrylique dans les compositions de la présente invention permettait d'obtenir sur les matières kératiniques, notamment les cheveux de très bonnes propriétés cosmétiques particulièrement au niveau de la légèreté, de la douceur, du lissage au toucher, au niveau de la souplesse et de la malléabilité sur cheveux séchés.

Il a aussi été constaté que les compositions de l'invention permettaient d'obtenir des cheveux séchés présentant au regard un aspect général plus lisse.

Il a encore été constaté que les associations selon l'invention présentaient une bonne tolérance cutanée.

L'invention a donc pour objet des compositions de lavage des matières kératiniques essentiellement caractérisées en ce qu'elles comprennent dans un milieu cosmétiquement acceptable:
i) au moins un agent tensio-actif détergent;
ii) au moins une silicone fonctionnalisée; et
iii) au moins un terpolymère acrylique constitué:
   - de 5 à 80% en poids, préférentiellement de 15 à 70% en poids et plus préférentiellement de 40 à 70% en poids, d'un monomère acrylate (a) choisi parmi un acrylate d'alkyle en C₁-C₆ et un méthacrylate d'alkyle en C₁-C₆;
   - de 5 à 80% en poids, préférentiellement de 10 à 70% en poids et plus préférentiellement de 20 à 60% en poids, d'un monomère (b) choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di-(C₁-C₄)alkylamino (C₁-C₄) alkyl (méth)acrylamide;
   - de 0,1 à 30% en poids, préférentiellement de 0,1 à 10% en poids d'un monomère (c) choisi parmi :
      un uréthane produit par réaction entre un isocyanate insaturé monoéthylénique et un agent tensioactif non ionique englobant un copolymère séquencé d'oxyde de 1,2-butylène et d'oxyde d'éthylène à extrémité alcoxy en C₁₋₄ ;
      un monomère tensioactif insaturé éthylénique copolymérisable obtenu par condensation d'un tensioactif non-ionique avec un acide carboxylique insaturé α,β-éthylénique ou son anhydride;
      un monomère tensioactif choisi parmi les produits de réaction de type urée d'un monoisocyanate insaturé monoéthylénique avec un tensioactif non-ionique présentant une fonction amine;
      un éther de (méth)allyle de formule CH₂=CR₁CH₂OAₘBₙAₚR₂ dans lequel R₁ désigne un atome d'hydrogène ou un groupe méthyle, A désigne un groupement propylèneoxy ou butylèneoxy, B désigne l'éthylèneoxy, n est égal à zéro ou désigne un nombre entier inférieur ou égal à 200 et préférentiellement inférieur ou égal à 100, m et p désignent zéro ou un nombre entier inférieur à n et R₂ est un groupe hydrophobe d'au moins 8 atomes de carbone et préférentiellement de 8 à 30 atomes de carbone; et
      un monomère non-ionique de type uréthane produit par réaction d'un tensioactif non ionique monohydrique avec un isocyanate insaturé monoéthylénique;
      les pourcentages en poids de monomères étant basés sur le poids total des monomères constituant le terpolymère.

Dans la composition de lavage de l'invention, le terpolymère acrylique est présent à raison de 0,01 à 20 % en poids de matières actives (M.A.) de préférence 0,1 à 10 % en poids par rapport au poids total de la composition.

Des monomères acrylates (a) préférés comprennent notamment les acrylates d'alkyle en C₂-C₆. L'acrylate d'éthyle est tout particulièrement préféré.

Comme exemples de monomères (b) préférés, il faut citer le méthacrylate de N,N-diméthylaminoéthyle (DMAEMA), l'acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropyle-méthacrylamide, le N,N-diéthylaminopropyl-acrylamide et le N,N-diéthylaminopropyl-méthacrylamide. Le méthacrylate de N,N-diméthylaminoéthyle est tout particulièrement préféré.

Les monomères (c) préférés sont les monomères tensio-actifs insaturés éthyléniques copolymérisables obtenus par condensation d'un tensioactif non-ionique avec un acide carboxylique insaturé α,β-éthylénique ou son anhydride, de préférence les acides mono ou di-carboxyliques en C₃-C₄ ou leurs anhydrides et plus particulièrement l'acide acrylique, l'acide méthacrylique, l'acide crotonique, l'acide maléique, l'anhydride maléique et tout particulièrement l'acide itaconique et l'anhydride itaconique.

Les monomères (c) particulièrement préférés correspondent aux monomères tensioactifs insaturés éthyléniques copolymérisables obtenus par condensation d'un tensioactif non-ionique avec l'acide itaconique. Parmi les tensioactifs non-ioniques, on peut citer notamment les alcools gras en C₁₀-C₃₀ alkoxylés avec 2 à 100, et de préférence de 5 à 50 moles d'oxyde d'alkylène, comme par exemple les éthers de polyéthylène glycol et d'alcools gras en C₁₀-C₃₀ et plus particulièrement les éthers de polyéthylène glycol et d'alcool cétylique, dénommés CETETH dans le dictionnaire CTFA, 7ème édition, 1997.

Des méthodes conventionnelles pour préparer ces terpolymères acryliques sont connues de l'homme du métier. De telles méthodes incluent la polymérisation en solution, la polymérisation par précipitation et la polymérisation en émulsion par exemple. Des terpolymères conformes à l'invention et leurs méthodes de préparation sont notamment décrits dans les demandes EP-A-0824914 et EP-A-0825200.

Parmi ces terpolymères, on préfère utiliser en particulier le polymère "STRUCTURE ® PLUS" vendu par la Société NATIONAL STARCH, qui est constitué d'acrylates, d'amino(méth)acrylates et d'itaconate d'alkyle en C₁₀-C₃₀ polyoxyéthyléné à 20 moles d'oxyde d'éthylène sous forme de dispersion aqueuse à 20% de M.A..

En plus de ces monomères, le terpolymère peut contenir d'autres monomères qui permettent de réticuler ledit terpolymère. Ces monomères sont utilisés dans des proportions assez faibles, jusqu'à 2% en poids par rapport au poids total des monomères utilisés pour préparer le terpolymère. De tels monomères de réticulation comprennent des monomères aromatiques portant plusieurs substituants vinyle, des monomères alicycliques portant plusieurs substituants vinyle, des esters bi-fonctionnels d'acide phtalique, des esters bi-fonctionnels d'acide méthacrylique, des esters multifonctionnels d'acide acrylique, le N-méthylène-bis-acrylamide et des monomères aliphatiques portant plusieurs substituants vinyle tels que des diènes, triènes et tétraènes. Des monomères de réticulation peuvent notamment être des divinyl-benzènes, des trivinyl-benzènes, des 1,2,4-trivinylcyclohexènes, des 1,5-hexadiènes, des 1,5,9-décatriènes, des 1,9-décadiènes, des 1,5-heptadiènes, des di-allyl phthalates, de l'éthylène glycol diméthacrylate, du polyéthylène glycol diméthacrylate, des penta- et tétra-acrylates, des triallyl pentaérythritols, des octaallyl saccharoses, des cycloparaffines, des cyclooléfines et du N-méthylène-bis-acrylamide.

Les silicones fonctionnalisées conformes à la présente invention sont des silicones comportant dans leur structure générale, un ou plusieurs groupements organofonctionnels directement fixés sur la chaîne siloxanique ou fixés par l'intermédiaire d'un radical hydrocarboné. Elles peuvent être utilisées telles quelles, sous forme d'émulsion ou de microémulsion.

On cite, par exemple, les silicones comportant:
a) des groupements polyéthylèneoxy et/ou polypropylèneoxy, comportant éventuellement des groupes alkyle, tels que
   - le produit dénommé diméthicone copolyol vendu par la Société DOW CORNING sous la dénomination "DC 1248", par la société UNION CARBIDE sous la dénomination huiles "SILWET" L 722, L 7500, L 77, L 711 et par la société RHODIA CHIMIE sous la dénomination "MIRASIL DMCO", l'alkyl (C12) méthicone copolyol vendu par la Société DOW CORNING sous la dénomination "Q2 5200"; et
   - le mélange de diméthicone copolyol et de cyclométhicone tel que le produit vendu sous la dénomination "Q2-3225C" par la Société DOW CORNING.
b) des groupements (per)fluorés comme les groupements trifluoroalkyle, telles que, par exemple, celles vendues par la Société GENERAL ELECTRIC sous les dénominations "FF.150 Fluorosilicone Fluid" ou pour la Société SHIN ETSU sous les dénomination "X-22-819"; "X-22-820"; X-22-821; "X-22-822"; ou "FL 100";
c) des groupements hydroxyacylamino, telles que celles décrites dans la demande de brevet européen EP-A-0 342 834 et en particulier la silicone vendue par la Société DOW CORNING sous la dénomination "Q2-8413" ;
d) des groupements thiols comme dans les silicones "X 2-8360" de DOW CORNING ou les "GP 72A" et "GP 71" de GENESEE ;
e) des groupements aminés substitués ou non, comme les produits commercialisés sous la dénomination GP 4 Silicone Fluid et GP 7100 par la société GENESEE ou les produits commercialisés sous les dénominations Q2 8220 et DC 929 ou 939 par la société DOW CORNING. Les groupements aminés substitués sont en particulier des groupements aminoalkyle en C₁-C₄ ou aminoalkyl(C₁-C₄)aminoalkyl(C₁-C₄). On utilise plus particulièrement les silicones dénommées amodiméthicone et triméthylsilylamodiméthicone selon la dénomination CTFA (1997);
f) des groupements carboxylates, comme les produits décrits dans le brevet européen EP 186 507 de CHISSO CORPORATION;
g) des groupements hydroxylés, comme les polyorganosiloxanes à fonction hydroxyalkyle, décrits dans la demande de brevet FR-A-2 589 476; et en particulier les polyorganosiloxanes à fonction γ-hydroxypropyle;
h) des groupements alcoxylés comportant au moins 12 atomes de carbone comme le produit "SILICONE COPOLYMER F 755" de SWS SILICONES et les produits "ABILWAX 2428", "ABILWAX 2434", "ABILWAX 2440" de la Société GOLDSCHMIDT;
i) des groupements acyloxyalkyle comportant au moins 12 atomes de carbone, comme par exemple les polyorganosiloxanes décrits dans la demande de brevet FR-A-2 641 185, et en particulier les polyorganosiloxanes à fonction stéaroyloxypropyle.
j) des groupements ammonium quaternaire, comme dans les produits "X2 81 08" et "X2 81 09", le produit "ABIL K 3270" de la Société GOLDSCHMIDT ;
k) des groupements amphotères ou bétaïniques, tels que dans le produit vendu par la Société GOLDSCHMIDT sous la dénomination "ABIL B 9950";
l) des groupements bisulfite, tels que dans les produits vendus par la Société GOLDSCHMIDT sous les dénominations "ABIL S201" et "ABIL S 255".

Parmi ces silicones, celles comportant des groupements aminés substitués ou non ou des groupements ammonium quaternaire ainsi que les diméthicones copolyols sont particulièrement préférées.

Les silicones fonctionnalisées peuvent être présentes dans des proportions comprises entre 0,01% et 20% en poids par rapport au poids total de la composition et préférentiellement dans des proportions comprises entre 0,1% et 10% en poids par rapport au poids total de la composition.

Comme indiqué précédemment, les compositions selon l'invention contiennent au moins un agent tensio-actif détergent, notamment choisi parmi les tensio-actifs anioniques, amphotères, non-ioniques et cationiques ayant des propriétés détergentes, et leurs mélanges.

Parmi les agents tensio-actifs anioniques, on peut citer les sels alcalins, les sels d'ammonium, les sels d'amines, les sels d'aminoalcools, les sels de magnésium des composés suivants : les alkylsulfates, alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates et les N-acyltaurates.

Le radical alkyle ou acyle de ces différents composés est généralement constitué par une chaîne carbonée comportant de 8 à 30 atomes de carbone.

Parmi les agents tensio-actifs anioniques, on peut également citer les sels d'acides gras tels que les sels des acides oléique, ricinoléique, palmitique, stéarique; les acides d'huile de coprah ou d'huile de coprah hydrogénée; les acyl lactylates, dont le radical acyle comporte de 8 à 30 atomes de carbone.

On peut également utiliser des agents tensio-actifs considérés comme faiblement anioniques tels que les acides alkyl ou alkylaryl éther carboxyliques polyoxyalkylénés ou leurs sels, les acides alkylamido éther carboxyliques polyoxyalkylénés ou leurs sels, les acides d'alkyl D-galactoside uroniques ou leurs sels.

Les agents tensio-actifs non-ioniques sont plus particulièrement choisis parmi les alcools ou les alkylphénols ou les acides gras polyéthoxylés, polypropoxylés ou polyglycérolés, à chaîne grasse comportant 8 à 30 atomes de carbone, le nombre de groupements oxyde d'éthylène ou oxyde de propylène étant compris entre 2 et 50 et le nombre de groupements glycérol étant compris entre 2 et 30.

On peut également citer les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amides gras polyglycérolés comportant de préférence 1 à 5 groupements glycérol et en particulier 1,5 à 4; les amines grasses polyéthoxylées ayant de préférence 2 à 30 moles d'oxyde d'éthylène; les esters d'acides gras du sorbitan oxyéthylénés avec 2 à 30 moles d'oxyde d'éthylène; les esters d'acide gras de sucrose, les esters d'acides gras du polyéthylèneglycol, les alkylpolyglycosides, les dérivés carbamates ou amides de N-alkyl glucamines, les aldobionamides, les oxydes d'amines tels que les oxydes d'alkylamines ou de N-acylamidopropyl-morpholine.

Les agents tensio-actifs amphotères préférés sont les dérivés d'amines secondaires ou tertiaires aliphatiques, dans lesquels le radical aliphatique est une chaîne linéaire ou ramifiée comportant 8 à 22 atomes de carbone et qui contient au moins un groupe anionique hydrosolubilisant, carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆) bétaïnes ou les alkyl(C₈-C₂₀)amidoalkyl(C₁-C₆)sulfobétaïnes.

Parmi les dérivés d'amines, on peut citer les produits vendus sous la dénomination MIRANOL, tels que décrits dans les brevets US-A-2 528 378 et 2 781 354 et classés dans le dictionnaire CTFA, 7ème édition, 1997, sous la dénomination Disodium Cocoamphodiacétate, Disodium Lauroamphodiacétate, Disodium Capryloamphodiacétate, Disodium Caproamphodiacétate, Disodium Cocoamphodipropionate, Disodium Lauroamphodipropionate, Disodium Caproamphodipropionate, Disodium Capryloamphodipropionate, Lauroamphodipropionate acide, Cocoamphodipropionate acide.

Les agents tensio-actifs cationiques sont notamment choisis parmi les sels d'amines grasses primaires, secondaires ou tertiaires, éventuellement polyoxyalkylénées; les sels d'ammonium quaternaire; les dérivés d'imidazoline; ou les oxydes d'amines à caractère cationique.

Les sels d'ammonium quaternaire préférés sont les halogénures (par ex. chlorures) de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore le chlorure de stéaramidopropyldiméthyl (myristyl acétate) ammonium commercialisé sous la dénomination «CEPHARYL 70» par la société VAN DYK.

On peut également utiliser les sels (chlorures ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol.

Les agents tensio-actifs sont utilisés dans les compositions conformes à l'invention dans des proportions suffisantes pour conférer un caractère détergent à la composition, généralement à raison d'au moins 4% en poids, de préférence entre 5 et 50% en poids par rapport au poids total de la composition et en particulier entre 8 et 35%.

Les compositions, selon l'invention, présentent un pH généralement compris entre 3 et 12, et plus particulièrement entre 4 et 8.

Le milieu cosmétiquement acceptable des compositions est constitué par de l'eau, par un ou plusieurs solvants ou par un mélange d'eau et d'au moins un solvant choisi parmi les alcools inférieurs, les alkylèneglycols et les éthers de polyol.

Les performances cosmétiques des compositions selon la présente invention peuvent être améliorées par ajout de polyorganosiloxanes différents des silicones fonctionnalisées décrites ci-dessus, et notamment les polyalkylsiloxanes tels que les polydiméthylsiloxanes linéaires à groupements terminaux triméthylsilyle, comme par exemple, et à titre non limitatif, les huiles "SILBIONE" de la série 70047 commercialisées par RHODIA CHIMIE; l'huile "47 V 500 000" de RHODIA CHIMIE, le "VISCASIL" de GENERAL ELECTRIC, ou le "MIRASIL" de RHODIA CHIMIE, le "DC 200" de viscosité 0,06 m².s⁻¹ de DOW CORNING ou l'"AK 300.000" de WACKER et les polydiméthylsiloxanes linéaires à groupements terminaux hydroxydiméthylsilyle, telles que les huiles de la série 48 V de RHODIA CHIMIE.

Les polyorganosiloxanes différents des silicones fonctionnalisées sont utilisés dans les compositions de l'invention dans des proportions bien connues de l'homme du métier.

Les performances cosmétiques des compositions de l'invention peuvent être améliorées aussi par addtion d'un polymère cationique choisi parmi tous ceux déjà connus en soi, notamment ceux décrits dans la demande de brevet EP-A-0 337 354 et dans les demandes de brevets français FR-A-2 270 846, 2 383 660, 2 598 611, 2 470 596 et 2 519 863.

Les polymères cationiques utilisés ont généralement une masse moléculaire comprise entre 500 et 5.10⁶ environ, et de préférence comprise entre 10³ et 3.10⁶ environ.

Parmi les polymères cationiques, on peut citer plus particulièrement les protéines (ou hydrolysats de protéines) quaternisées et les polymères du type polyamine, polyaminoamide et polyammonium quaternaire. Ce sont des produits connus.

Les polymères du type polyamine, polyaminoamide, polyammonium quaternaire, utilisables conformément à la présente invention, pouvant être notamment mentionnés, sont ceux décrits dans les brevets français n°2 505 348 ou 2 542 997.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi les polymères cationiques susceptibles d'être utilisés dans le cadre de la présente invention, on préfère les dérivés d'éthers de cellulose comportant des groupements ammonium quaternaires, les polysaccharides et notamment gommes de guar cationiques et les cyclopolymères de méthyl diallyl amine ou de diméthyl diallyl ammonium.

Les polymères cationiques sont utilisés dans les compositions de l'invention dans des proportions comprises entre 0,001 et 20% en poids et de préférence entre 0,05 et 5% en poids par rapport au poids total de la composition.

Les compositions selon l'invention peuvent également contenir en outre au moins un adjuvant choisi parmi les adjuvants habituellement utilisés en cosmétique, tel que les parfums, les conservateurs, les séquestrants, les humectants, les sucres, les huiles végétales, minérales, animales ou de synthèse, les polymères amphotères, le menthol, les dérivés de nicotinate, les agents anti-chute des cheveux, les agents anti-pelliculaires, les stabilisateurs de mousse, les agents propulseurs, les filtres, les colorants, les céramides, les vitamines ou provitamines, les agents acidifiants ou alcalinisants ou d'autres adjuvants cosmétiques bien connus.

Dans une forme de réalisation préférée de l'invention, les compositions selon l'invention sont utilisées comme shampooings pour le lavage des cheveux.

Le procédé de lavage des matières kératiniques consiste à appliquer une composition telle que définie ci-dessus sur des matières kératiniques humides ou sèches dans des quantités efficaces pour les laver, cette application étant suivie d'un rinçage après un temps de pose facultatif.

L'exemple qui suit est destiné à illustrer l'invention.

### EXEMPLE DE SHAMPOOING

| | |
|---|---|
| Propylène glycol | 0,1 g |
| Cocoyl bétaïne en solution aqueuse à 30% | 10 g |
| Chlorure d'hydroxypropyl guar triméthyl ammonium vendu par la société MEYHALL sous la dénomination "JAGUAR C13S" | 0,1 g |
| Mélange 1-(Hexadécyloxy)-2-octadécanol / alcool cétylique | 2,5 g |
| monoisopropanolamide d'acides de coprah | 0,6 g |
| Polydiméthylsiloxane à groupements aminoéthyl iminopropyl en émulsion cationique à 35% dans l'eau vendu par la société DOW CORNING sous la dénomination "DC 939" | 7 g |
| Lauryl éther sulfate de sodium (2,2 OE) à environ 70% de M.A. | 22 g |
| Terpolymère d'acrylates, d'amino(méth)acrylates et d'itaconate d'alkyl en C₁₀-C₃₀ polyoxyéthyléné à 20 moles d'oxyde d'éthylène sous forme de dispersion aqueuse à 20% de M.A. vendu par la société National Starch sous la dénomination "STRUCTURE® PLUS" | 1 g |
| Conservateurs | qs |
| Eau déminéralisée stérilisée | qsp 100 g |

On ajuste le pH à 5 par de l'acide citrique.

Après lavage avec ce shampooing, il a été constaté que les cheveux séchés étaient doux, souples, lisses au toucher et au regard, et présentaient une bonne malléabilité.

## Revendications

1. Composition de lavage des matières kératiniques, comprenant dans un milieu cosmétiquement acceptable, au moins un agent tensio-actif détergent et au moins une silicone fonctionnalisée, **caractérisée par le fait qu'**elle comprend en outre au moins un terpolymère acrylique constitué:
- de 5 à 80% en poids, préférentiellement de 15 à 70% en poids et plus préférentiellement de 40 à 70% en poids, d'un monomère acrylate (a) choisi parmi un acrylate d'alkyle en C₁-C₆ et un méthacrylate d'alkyle en C₁-C₆;
- de 5 à 80% en poids, préférentiellement de 10 à 70% en poids et plus préférentiellement de 20 à 60% en poids, d'un monomère (b) choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di (C₁-C₄)alkylamino (C₁-C₄) alkyl (méth)acrylamide;
- de 0,1 à 30% en poids, préférentiellement de 0,1 à 10% en poids d'un monomère (c) choisi parmi :
un uréthane produit par réaction entre un isocyanate insaturé monoéthylénique et un agent tensioactif non ionique englobant un copolymère séquencé d'oxyde de 1,2-butylène et d'oxyde d'éthylène à extrémité alcoxy en C₁₋₄ ;
un monomère tensioactif insaturé éthylénique copolymérisable obtenu par condensation d'un tensioactif non-ionique avec un acide carboxylique insaturé α,β-éthylénique ou son anhydride;
un monomère tensioactif choisi parmi les produits de réaction de type urée d'un monoisocyanate insaturé monoéthylénique avec un tensioactif non ionique présentant une fonctionalité amine;
un éther de (meth)allyle de formule CH₂=CR₁CH₂OAₘBₙAₚR₂ dans lequel R₁ désigne un atome d'hydrogène ou un groupe méthyle, A désigne un groupement propylèneoxy ou butylèneoxy, B désigne éthylèneoxy,
n est égal à zéro ou désigne un nombre entier inférieur ou égal à 200,
m et p désignent zéro ou un nombre entier inférieur à n et R₂ est un groupe hydrophobe d'au moins 8 atomes de carbone; et
un monomère non-ionique de type uréthane produit par réaction d'un tensioactif non ionique monohydrique avec un isocyanate insaturé monoéthylénique;
les pourcentages en poids de monomères étant basés sur le poids total des monomères constituant le terpolymère.

2. Composition selon la revendication 1, **caractérisée en ce que** le terpolymère est présent à raison de 0,01 à 20% en poids de matière active, de préférence 0,1 à 10 % en poids par rapport au poids total de la composition.

3. Composition selon la revendication 1 ou 2, **caractérisée en ce que** le monomère (a) est choisi parmi les acrylates d'alkyle en C₂-C₆, et est préférentiellement l'acrylate d'éthyle.

4. Composition selon l'une quelconque des revendications 1 à 3, **caractérisée en ce que** le monomère (b) est choisi parmi le méthacrylate de N,N-diméthylaminoéthyle, l'acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropyl-méthacrylamide, le N,N-diéthylaminopropyl-acrylamide et le N,N-diéthylaminopropyl-méthacrylamide, et est préférentiellement le méthacrylate de N,N-diméthylaminoéthyle.

5. Composition selon l'une quelconque des revendications 1 à 4, **caractérisée en ce que** le monomère (c) est un monomère tensioactif insaturé éthylénique copolymérisable obtenu par condensation d'un tensioactif non-ionique avec l'acide itaconique.

6. Composition selon l'une quelconque des revendications 1 à 5, **caractérisée en ce que** le terpolymère acrylique est constitué d'acrylates, d'amino(méth)acrylates et d'itaconate d'alkyle en C₁₀-C₃₀ polyoxyéthyléné à 20 moles d'oxyde d'éthylène.

7. Composition selon l'une quelconque des revendications 1 à 6, **caractérisée en ce que** le terpolymère acrylique contient en outre un monomère de réticulation.

8. Composition selon l'une quelconque des revendications 1 à 7, **caractérisée en ce que** la silicone fonctionnalisée est choisie parmi les polyorganosiloxanes organomodifiés, comportant dans leur structure générale, un ou plusieurs groupements organofonctionnels directement fixés sur la chaîne siloxanique ou fixés par l'intermédiaire d'un radical hydrocarboné.

9. Composition selon l'une quelconque des revendications 1 à 8, **caractérisée en ce que** la silicone fonctionnalisée est choisie parmi les silicones comportant:
a) des groupements polyéthylèneoxy et/ou polypropylèneoxy, comportant éventuellement des groupes alkyle;
b) des groupements (per)fluorés comme les groupements trifluoroalkyle;
c) des groupements hydroxyacylamino;
d) des groupements thiols;
e) des groupements aminés substitués ou non;
f) des groupements carboxylates;
g) des groupements hydroxylés, comme les polyorganosiloxanes à fonction hydroxyalkyle, en particulier les polyorganosiloxanes à fonction γ-hydroxypropyle;
h) des groupements alcoxylés comportant au moins 12 atomes de carbone;
i) des groupements acyloxyalkyle comportant au moins 12 atomes de carbone, en particulier les polyorganosiloxanes à fonction stéaroyloxypropyle;
j) des groupements ammonium quaternaire;
k) des groupements amphotères ou bétaïniques; ou
l) des groupements bisulfite.

10. Composition selon l'une quelconque des revendications 1 à 9, **caractérisée en ce que** la silicone fonctionnalisée est choisie parmi les silicones comportant des groupements aminés substitués ou non ou des groupements ammonium quaternaire.

11. Composition selon l'une quelconque des revendications 1 à 10, **caractérisée par le fait que** la silicone fonctionnalisée est présente à raison de 0,01 à 20% en poids, de préférence de 0,1 à 10% en poids par rapport au poids total de la composition.

12. Composition selon l'une quelconque des revendications 1 à 11, **caractérisée en ce que** l'agent tensio-actif détergent est choisi parmi les agents tensio-actifs anioniques, amphotères, non-ioniques, cationiques et leurs mélanges.

13. Composition selon la revendication 12, **caractérisée par le fait que** les agents tensio-actifs anioniques sont choisis parmi les sels alcalins, les sels de magnésium, les sels d'ammonium, les sels d'amines ou les sels d'aminoalcools des composés suivants : les alkylsulfates, alkyléthersulfates, alkylamidoéthersulfates, alkylarylpolyéthersulfates, monoglycérides sulfates; les alkylsulfonates, alkylamides sulfonates, alkylaryl-sulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates, N-acyltaurates; le radical alkyle ou acyle de ces différents composés étant constitué par une chaîne carbonée comportant de 8 à 30 atomes de carbone; les sels d'acides gras des acides oléique, ricinoléique, palmitique, stéarique; les acides d'huile de coprah ou d'huile de coprah hydrogénée; les acyl lactylates, dont le radical acyle comporte de 8 à 30 atomes de carbone; les acides d'alkyl D-galactoside uroniques et leurs sels, les acides alkyl ou alkylaryl éther carboxyliques polyoxyalkylénés ou leurs sels, les acides alkylamido éther carboxyliques polyoxyalkylénés ou leurs sels.

14. Composition selon la revendication 12, **caractérisée par le fait que** les agents tensio-actifs non-ioniques sont choisis parmi les alcools ou les alkylphénols ou les acides gras polyéthoxylés, polyoxypropylénés ou polyglycérolés, à chaîne grasse comportant 8 à 30 atomes de carbone, le nombre de groupements oxyde d'éthylène, oxyde de propylène étant compris entre 2 et 50 et le nombre de groupements glycérol étant compris entre 2 et 30; les copolymères d'oxyde d'éthylène et de propylène; les condensats d'oxyde d'éthylène et de propylène sur des alcools gras; les amides gras polyéthoxylés; les amides gras polyglycérolés; les amines grasses polyéthoxylées; les esters d'acides gras du sorbitan oxyéthylénés; les esters d'acides gras de sucrose ou du polyéthylèneglycol; les alkylpolyglycosides; les dérivés amides ou carbamates de N-alkylglucamines, les aldobionamides et les oxydes d'amines.

15. Composition selon la revendication 12, **caractérisée par le fait que** les agents tensio-actifs amphotères sont choisis parmi les dérivés d'amines secondaires ou tertiaires aliphatiques, dans lesquels le radical aliphatique est une chaîne linéaire ou ramifiée comportant de 8 à 22 atomes de carbone et qui contient au moins un groupe anionique hydrosolubilisant, carboxylate, sulfonate, sulfate, phosphate, phosphonate; les alkyl(C₈-C₂₀)bétaïnes, les sulfobétaïnes, les alkyl(C₈-C₂₀)amidoalkyl(C₁-C₆) bétaïnes ou les alkyl(C₈-C₂₀)amidoalkyl(C₁-C₆)sulfobétaïnes.

16. Composition selon la revendication 11, **caractérisée par le fait que** les agents tensio-actifs cationiques sont choisis parmi les sels d'ammonium quaternaire et préférentiellement parmi le chlorure de dilauryldiméthyl ammonium, le chlorure de diisobutyl phénoxy éthoxy éthyl diméthylbenzyl ammonium, le bromure de cétyl triméthyl ammonium, le bromure de N-cétyl pyridinium et le chlorure de benzéthonium.

17. Composition selon l'une quelconque des revendications 1 à 16, **caractérisée en ce que** l'agent tensio-actif détergent est présent à raison d'au moins 4% en poids, de préférence de 5 à 50% en poids, et encore plus préférentiellement de 8 à 35% en poids par rapport au poids total de la composition.

18. Composition selon l'une quelconque des revendications 1 à 17, **caractérisée en ce qu'**elle présente un pH compris entre 3 et 12, et plus particulièrement entre 4 et 8.

19. Composition selon l'une quelconque des revendications 1 à 18, **caractérisée par le fait que** le milieu cosmétiquement acceptable est constitué par de l'eau, par un ou plusieurs solvants ou par un mélange d'eau et d'au moins un solvant choisi parmi les alcools inférieurs, les alkylèneglycols et les éthers de polyol.

20. Composition selon l'une quelconque des revendications 1 à 19, **caractérisée par le fait qu'**elle contient en outre au moins un polyorganosiloxane différent des silicones fonctionnalisées telles que définies dans les revendications 8 à 11, de préférence choisi parmi les polydiméthylsiloxanes linéaires à groupements terminaux triméthylsilyle ou hydroxydiméthylsilyle.

21. Composition selon l'une quelconque des revendications 1 à 20, **caractérisée par le fait qu'**elle contient en outre au moins un polymère cationique.

22. Composition selon la revendication 21, **caractérisée par le fait que** le polymère cationique est choisi parmi les dérivés d'éthers de cellulose comportant des groupements ammonium quaternaires, les polysaccharides et notamment gommes de guar cationiques et les cyclopolymères de méthyl diallyl amine ou de diméthyl diallyl ammonium.

23. Composition selon la revendication 22, **caractérisée par le fait qu'**elle contient un polymère cationique dans des proportions comprises entre 0,001 et 20% en poids et de préférence entre 0,05 et 5% en poids, par rapport au poids total de la composition.

24. Composition selon l'une quelconque des revendications 1 à 23, **caractérisée par le fait qu'**elle contient en outre au moins un adjuvant cosmétiquement acceptable choisi parmi les parfums, les conservateurs, les séquestrants, les humectants, les sucres, les huiles végétales, minérales, animales ou de synthèse, les polymères amphotères, le menthol, les dérivés de nicotinate, les agents anti-chute des cheveux, les agents anti-pelliculaires, les stabilisateurs de mousse, les agents propulseurs, les filtres, les colorants, les céramides, les vitamines ou provitamines et les agents acidifiants ou alcalinisants.

25. Utilisation comme shampooing de la composition telle que définie dans l'une quelconque des revendications 1 à 24.

26. Procédé de lavage des matières kératiniques, **caractérisé par le fait que** l'on applique sur les matières kératiniques humides ou sèches au moins une composition telle que définie dans l'une quelconque des revendications 1 à 24, et après un temps de pose facultatif, on les rince à l'eau.

## Patentansprüche

1. Zusammensetzung zur Reinigung von Keratinsubstanzen, die in einem kosmetisch akzeptablen Medium mindestens einen reinigenden grenzflächenaktiven Stoff und mindestens ein funktionalisiertes Silicon enthält, **dadurch gekennzeichnet, daß** sie ferner mindestens ein Acrylterpolymer enthält, das besteht aus:
- 5 bis 80 Gew.-%, vorzugsweise 15 bis 70 Gew.-% und noch bevorzugter 40 bis 70 Gew.-% eines Acrylatmonomers (a), das unter den C₁₋₆-Alkylacrylaten und C₁₋₆-Alkylmethacrylaten ausgewählt ist;
- 5 bis 80 Gew.-%, vorzugsweise 10 bis 70 Gew.-% und noch bevorzugter 20 bis 60 Gew.-% eines Monomers (b), das unter den heterocyclischen Vinylverbindungen, die mindestens ein Stickstoffatom oder Schwefelatom enthalten, (Meth)acrylamiden, Mono- oder Di-C₁₋₄-alkylamino-C₁₋₄-alkyl(meth)-acrylaten und Mono- oder Di-C₁₋₄-Alkylamino-C₁₋₄-alkyl(meth)acrylamiden ausgewählt ist;
- 0,1 bis 30 Gew.-% und vorzugsweise 0,1 bis 10 Gew.-% eines Monomers (c), das ausgewählt ist unter:
· Urethanen, die bei der Umsetzung eines monoethylenisch ungesättigten Isocyanats mit einem nichtionischen grenzflächenaktiven Stoff, der ein Sequenzcopolymer von 1,2-Butylenoxid und Ethylenoxid mit endständiger C₁₋₄-Alkoxygruppe einschließt, entsteht;
· copolymerisierbaren, ethylenisch ungesättigten Tensidmonomeren, die bei der Kondensation von nichtionischen grenzflächenaktiven Stoffen mit α,β-ethylenisch ungesättigten Carbonsäuren oder ihren Anhydriden gebildet werden;
· Tensidmonomeren, die unter den Reaktionsprodukten vom Harnstofftyp von monoethylenisch ungesättigten Monoisocyanaten mit nichtionischen grenzflächenaktiven Stoffen, die eine Aminogruppe aufweisen, ausgewählt sind;
· (Meth)allylethern der Formel CH₂=CR₁CH₂OAₘBₙAₚR₂, wobei in der Formel R₁ ein Wasserstoffatom oder die Methylgruppe bedeutet, A eine Propylenoxy- oder Butylenoxygruppe bezeichnet, B Ethylenoxy ist, n Null oder eine ganze Zahl von höchstens 200 und m und p Null oder eine ganze Zahl unter n bedeuten und R₂ eine hydrophobe Gruppe mit mindestens 8 Kohlenstoffatomen ist; und
· nichtionischen Monomeren vom Urethantyp, die bei der Reaktion von nichtionischen grenzflächenaktiven Stoffen mit einer Hydroxygruppe und monoethylenisch ungesättigten Isocyanaten gebildet werden;
wobei die prozentualen Gewichtsanteile der Monomere auf dem Gesamtgewicht der Monomere, die das Terpolymer bilden, basieren.

2. Zusammensetzung nach Anspruch 1, **dadurch gekennzeichnet, daß** das Terpolymer in einer Menge von 0,01 bis 20 Gew.-% Wirkstoff und vorzugsweise 0,1 bis 10 Gew.-%, bezogen auf das Gesamtgewicht der Zusammensetzung, vorliegt.

3. Zusammensetzung nach Anspruch 1 oder 2, **dadurch gekennzeichnet, daß** das Monomer (a) unter den C₂₋₆-Alkylacrylaten ausgewählt ist und es sich vorzugsweise um Ethylacrylat handelt.

4. Zusammensetzung nach einem der Ansprüche 1 bis 3, **dadurch gekennzeichnet, daß** das Monomer (b) unter N,N-Dimethylaminoethylmethacrylat, N,N-Diethylaminoethylacrylat, N,N-Diethylaminoethylmethacrylat, N-*t*-Butylaminoethylacrylat, N-*t*-Butylaminoethylmethacrylat, N,N-Dimethylaminopropylacrylamid, N,N-Dimethylaminopropylmethacrylamid, N,N-Diethylaminopropylacrylamid und N,N-Diethylaminopropylmethacrylamid und vorzugsweise dem N,N-Dimethylaminoethylmethacrylat ausgewählt ist.

5. Zusammensetzung nach einem der Ansprüche 1 bis 4, **dadurch gekennzeichnet, daß** das Monomer (c) ein copolymerisierbares, ethylenisch ungesättigtes Tensidmonomer ist, das durch Kondensation eines nichtionischen grenzflächenaktiven Stoffes mit Itaconsäure entsteht.

6. Zusammensetzung nach einem der Ansprüche 1 bis 5, **dadurch gekennzeichnet, daß** das Acrylterpolymer aus Acrylaten, Amino(meth)acrylaten und mit 20 mol Ethylenoxid polyethoxyliertem C₁₀₋₃₀-Alkylitaconat besteht.

7. Zusammensetzung nach einem der Ansprüche 1 bis 6, **dadurch gekennzeichnet, daß** das Acrylterpolymer ferner ein Monomer zur Vernetzung enthält.

8. Zusammensetzung nach einem der Ansprüche 1 bis 7, **dadurch gekennzeichnet, daß** das funktionalisierte Silicon unter den organomodifizierten Polyorganosiloxanen ausgewählt ist, die in ihrer allgemeinen Struktur eine oder mehrere organofunktionelle Gruppen enthalten, die direkt an die Siloxankette oder über eine Kohlenwasserstoffgruppe an die Siloxankette gebunden sind.

9. Zusammensetzung nach einem der Ansprüche 1 bis 8, **dadurch gekennzeichnet, daß** das funktionalisierte Silicon unter den Siliconen ausgewählt ist, die enthalten:
a) Polyethylenoxy- und/oder Polypropylenoxygruppen, die gegebenenfalls Alkylgruppen aufweisen;
b) (per)fluorierte Gruppen, beispielsweise Trifluoralkylgruppen;
c) Hydroxyacylaminogruppen;
d) Thiolgruppen;
e) substituierte oder unsubstituierte aminierte Gruppen;
f) Carboxylatgruppen;
g) hydroxylierte Gruppen, beispielsweise die Polyorganosiloxane mit Hydroxyalkylgruppe und insbesondere Polyorganosiloxane mit γ-Hydroxypropylgruppe;
h) alkoxylierte Gruppen mit mindestens 12 Kohlenstoffatomen;
i) Acyloxyalkylgruppen mit mindestens 12 Kohlenstoffatomen und insbesondere Polyorganosiloxane mit Stearoyloxypropylgruppe;
j) quartäre Ammoniumgruppen;
k) amphotere Gruppen oder Betaingruppen;
l) Bisulfitgruppen.

10. Zusammensetzung nach einem der Ansprüche 1 bis 9, **dadurch gekennzeichnet, daß** funktionalisierte Silicon unter den Siliconen ausgewählt ist, die substituierte oder unsubstituierte aminierte Gruppen oder quartäre Ammoniumgruppen tragen.

11. Zusammensetzung nach einem der Ansprüche 1 bis 10, **dadurch gekennzeichnet, daß** das funktionalisierte Silicon in einer Menge von 0,01 bis 20 Gew.-% und vorzugsweise 0,2 bis 10 Gew.-%, bezogen auf das Gesamtgewicht der Zusammensetzung, vorliegt.

12. Zusammensetzung nach einem der Ansprüche 1 bis 11, **dadurch gekennzeichnet, daß** der reinigende grenzflächenaktive Stoff unter den anionischen, amphoteren, nichtionischen und kationischen grenzflächenaktiven Stoffen und deren Gemischen ausgewählt ist.

13. Zusammensetzung nach Anspruch 12, **dadurch gekennzeichnet, daß** die anionischen grenzflächenaktiven Stoffe unter den Alkalisalzen, Magnesiumsalzen, Ammoniumsalzen, Aminsalzen oder Aminoalkoholsalzen der folgenden Verbindungen: Alkylsulfate, Alkylethersulfate, Alkylamidoethersulfate, Alkylarylpolyethersulfate, Monoglyceridsulfate; Alkylsulfonate, Alkylamidsulfonate, Alkylarylsulfonate, Olefinsulfonate, Paraffinsulfonate; Alkylsulfosuccinate, Alkylethersulfosuccinate, Alkylamidsulfosuccinate; Alkylsulfosuccinamate; Alkylsulfoacetate; Alkylphosphate, Alkyletherphosphate; Acylsarcosinate, Acylisethionate und N-Acyltaurate; wobei die Alkyl- oder Acylgruppen dieser verschiedenen Verbindungen aus einer Kohlenstoffkette mit 8 bis 30 Kohlenstoffatomen bestehen; Salzen von Ölsäure, Ricinolsäure, Palmitinsäure und Stearinsäure; Säuren von Kopraöl oder hydriertem Kopraöl; Acyllactylaten, deren Acylgruppe 8 bis 30 Kohlenstoffatome aufweist; Alkyl-D-galactosiduronsäuren und ihren Salzen, polyalkoxylierten Alkyl- oder Alkylarylethercarbonsäuren und ihren Salzen, polyalkoxylierten Alkylamidoethercarbonsäuren und ihren Salzen ausgewählt sind.

14. Zusammensetzung nach Anspruch 12, **dadurch gekennzeichnet, daß** die nichtionischen grenzflächenaktiven Stoffe unter den Alkoholen oder Alkylphenolen oder Fettsäuren, die polyethoxyliert, polypropoxyliert oder mehrfach mit Glycerin verethert sind und eine Fettkette mit 8 bis 30 Kohlenstoffatomen aufweisen, wobei die Zahl der Ethylenoxid- und Propylenoxidgruppen im Bereich von 2 bis 50 und die Anzahl der Glyceringruppen im Bereich von 2 bis 30 liegt; den Copolymeren von Ethylenoxid und Propylenoxid; den Kondensaten von Ethylenoxid und Propylenoxid mit Fettalkoholen; polyethoxylierten Fettamiden; mehrfach mit Glycerin veretherten Fettamiden; polyethoxylierten Fettaminen; ethoxylierten Sorbitanfettsäureestern; Saccharosefettsäureestern oder Polyethylenglykolfettsäureestern; Alkylpolyglykosiden; Amid- oder Carbamatderivaten von N-Alkylglucaminen, Aldobionamiden und Aminoxiden ausgewählt sind.

15. Zusammensetzung nach Anspruch 12, **dadurch gekennzeichnet, daß** die amphoteren grenzflächenaktiven Stoffe unter den aliphatischen, sekundären oder tertiären Aminderivaten, wobei die aliphatische Gruppe eine lineare oder verzweigte Kette mit 8 bis 22 Kohlenstoffatomen bedeutet, die mindestens eine wasserlösliche anionische Gruppe, Carboxylat, Sulfonat, Sulfat, Phosphat oder Phosphonat aufweist; C₈₋₂₀-Alkylbetainen; Sulfobetainen, C₈₋₂₀-Alkyl-C₁₋₆-amidoalkylbetainen oder C₈₋₂₀-Alkyl-C₁₋₆-amidoalkylsulfobetainen ausgewählt sind.

16. Zusammensetzung nach Anspruch 11, **dadurch gekennzeichnet, daß** die kationischen grenzflächenaktiven Stoffe unter den quartären Ammoniumsalzen und vorzugsweise unter Dilauryldimethylammoniumchlorid, Diisobutylphenoxyethoryethyldimethylbenzylammoniumchlorid, Cetyltrimethylammoniumbromid, N-Cetylpyridiniumbromid und Benzethoniumchlorid ausgewählt sind.

17. Zusammensetzung nach einem der Ansprüche 1 bis 16, **dadurch gekennzeichnet, daß** der reinigende grenzflächenaktive Stoff in einer Menge von mindestens 4 Gew.-%, vorzugsweise 5 bis 50 Gew.-% und besonders bevorzugt 8 bis 35 Gew.-%, bezogen auf das Gesamtgewicht der Zusammensetzung, vorliegt.

18. Zusammensetzung nach einem der Ansprüche 1 bis 17, **dadurch gekennzeichnet, daß** sie einen pH-Wert im Bereich von 3 bis 12 und insbesondere 4 bis 8 aufweist.

19. Zusammensetzung nach einem der Ansprüche 1 bis 18, **dadurch gekennzeichnet, daß** das kosmetisch akzeptable Medium aus Wasser oder einem oder mehreren Lösungsmitteln oder einem Gemisch von Wasser und mindestens einem kosmetisch akzeptablen Lösungsmittel besteht, das unter den niederen Alkoholen, Alkylenglykolen und Polyolethern ausgewählt ist.

20. Zusammensetzung nach einem der Ansprüche 1 bis 19, **dadurch gekennzeichnet, daß** sie ferner mindestens ein Polyorganosiloxan enthält, das von den funktionalisierten Siliconen nach Anspruch 8 bis 11 verschieden ist und das vorzugsweise unter den linearen Polydimethylsiloxanen mit endständigen Trimethylsilyl- oder Hydroxydimethylsilylgruppen ausgewählt ist

21. Zusammensetzung nach einem der Ansprüche 1 bis 20, **dadurch gekennzeichnet, daß** sie außerdem mindestens ein kationisches Polymer enthält.

22. Zusammensetzung nach Anspruch 21, **dadurch gekennzeichnet, daß** das kationische Polymer unter den Celluloseetherderivaten mit quartären Ammoniumgruppen, Polysacchariden und insbesondere kationischen Guargummen und Methyldiallylamin-Cyclopolymeren oder Dimethyldiallyammonium-Cyclopolymeren ausgewählt ist.

23. Zusammensetzung nach Anspruch 22, **dadurch gekennzeichnet, daß** sie ein kationisches Polymer in Mengenanteilen im Bereich von 0,001 bis 20 Gew.-% und vorzugsweise 0,05 bis 5 Gew.-%, bezogen auf das Gesamtgewicht der Zusammensetzung, enthält.

24. Zusammensetzung nach einem der Ansprüche 1 bis 23, **dadurch gekennzeichnet, daß** sie außerdem mindestens einen kosmetisch akzeptablen Zusatzstoff enthält, der ausgewählt ist unter: Parfums, Konservierungsmitteln, Maskierungsmitteln, Feuchthaltemitteln, Zuckern, pflanzlichen, mineralischen, tierischen oder synthetischen Ölen, amphoteren Polymeren, Menthol, Nicotinatderivaten, Wirkstoffen gegen Haarausfall, Wirkstoffen gegen Schuppen, Schaumstabilisatoren, Treibmitteln, Filtern, Farbmitteln, Ceramiden, Vitaminen oder Provitaminen und Ansäuerungs- oder Alkalisierungsmitteln.

25. Verwendung der Zusammensetzung nach einem der Ansprüche 1 bis 24 als Haarwaschmittel.

26. Verfahren zur Reinigung von Keratinsubstanzen, **dadurch gekennzeichnet, daß** auf die feuchten oder trockenen Keratinsubstanzen mindestens-eine Zusammensetzung nach einem der Ansprüche 1 bis 24 aufgetragen und nach einer fakultativen Einwirkzeit mit Wasser gespült wird.

## Claims

1. Composition for washing keratin materials, comprising, in a cosmetically acceptable medium, at least one detergent surfactant and at least one functionalized silicone, **characterized in that** it also comprises at least one acrylic terpolymer consisting of:
- from 5% to 80% by weight, preferably from 15% to 70% by weight and more preferably from 40% to 70% by weight, of an acrylate monomer (a) chosen from a C₁-C₆ alkyl acrylate and a C₁-C₆ alkyl methacrylate;
- from 5% to 80% by weight, preferably from 10% to 70% by weight and more preferably from 20% to 60% by weight, of a monomer (b) chosen from a heterocyclic vinyl compound containing at least one nitrogen or sulphur atom, a (meth)acrylamide, a mono- or di(C₁-C₄)alkylamino(C₁-C₄)alkyl (meth)acrylate and a mono- or di(C₁-C₄)alkylamino(C₁-C₄)alkyl(meth)acrylamide;
- from 0.1% to 30% by weight, preferably from 0.1% to 10% by weight, of a monomer (c) chosen from:
a urethane produced by reaction between a monoethylenic unsaturated isocyanate and a nonionic surfactant encompassing a block copolymer of 1,2-butylene oxide and of ethylene oxide containing a C₁₋₄ alkoxy end;
a copolymerizable ethylenic unsaturated surfactant monomer obtained by condensing a nonionic surfactant with an α,β-ethylenic unsaturated carboxylic acid or its anhydride;
a surfactant monomer chosen from reaction products such as urea of a monoethylenic unsaturated monoisocyanate with a nonionic surfactant containing an amine function;
a (meth)allyl ether of formula CH₂=CR₁CH₂OAₘBₙAₚR₂ in which R₁ denotes a hydrogen atom or a methyl group, A denotes a propylenoxy or butylenoxy group, B denotes ethylenoxy,
n is equal to zero or denotes an integer less than or equal to 200, m and p denote zero or an integer less than n and R₂ is a hydrophobic group of at least 8 carbon atoms and preferably of C₈-C₃₀; and
a nonionic monomer such as urethane produced by reaction of a monohydric nonionic surfactant with a monoethylenic unsaturated isocyanate;
the weight percentages of monomers being based on the total weight of the monomers constituting the terpolymer.

2. Composition according to Claim 1, **characterized in that** the terpolymer is present in a proportion of from 0.01% to 20% by weight of active material, preferably 0.1% to 10% by weight, relative to the total weight of the composition.

3. Composition according to Claim 1 or 2, **characterized in that** the monomer (a) is chosen from C₂-C₆ alkyl acrylates and is preferably ethyl acrylate.

4. Composition according to any one of Claims 1 to 3, **characterized in that** the monomer (b) is chosen from N,N-dimethylaminoethyl methacrylate, N,N-diethylaminoethyl acrylate, N,N-diethylaminoethyl methacrylate, N-t-butylaminoethyl acrylate, N-t-butylaminoethyl methacrylate, N,.N-dimethylaminopropylacrylamide, N,N-dimethylaminopropylmethacrylamide, N,N-diethylaminopropylacrylamide and N,N-diethyl-aminopropylmethacrylamide and is preferably N,N-dimethyl-aminoethyl methacrylate.

5. Composition according to any one of Claims 1 to 4, **characterized in that** the monomer (c) is a copolymerizable ethylenic unsaturated surfactant monomer obtained by condensing a nonionic surfactant with itaconic acid.

6. Composition according to any one of Claims 1 to 5, **characterized in that** the acrylic terpolymer consists of acrylates, amino(meth)acrylates and itaconate C₁₀-C₃₀ alkyl; polyoxyethylenated with 20 mol of ethylene oxide.

7. Composition according to any one of Claims 1 to 6, **characterized in that** the acrylic terpolymer also contains a crosslinking monomer.

8. Composition according to any one of Claims 1 to 7, **characterized in that** the functionalized silicone is chosen from organomodified polyorganosiloxanes comprising in their general structure one or more organofunctional groups directly attached to the siloxane chain or attached via a hydrocarbon-based radical.

9. Composition according to any one of Claims 1 to 8, **characterized in that** the functionalized silicone is chosen from silicones comprising:
a) polyethylenoxy and/or polypropylenoxy groups, optionally comprising alkyl groups;
b) (per)fluoro groups, for instance trifluoroalkyl groups;
c) hydroxyacylamino groups;
d) thiol groups;
e) substituted or unsubstituted amine groups;
f) carboxylate groups;
g) hydroxyl groups, for instance polyorganosiloxanes containing a hydroxyalkyl function, in particular polyorganosiloxanes containing a γ-hydroxypropyl function;
h) alkoxy groups containing at least 12 carbon atoms;
i) acyloxyalkyl groups containing at least 12 carbon atoms, in particular polyorganosiloxanes containing a stearoyloxypropyl function;
j) quaternary ammonium groups;
k) amphoteric or betaine groups; or
l) bisulphite groups.

10. Composition according to any one of Claims 1 to 9, **characterized in that** the functionalized silicone is chosen from silicones comprising substituted or unsubstituted amine groups or quaternary ammonium groups.

11. Composition according to any one of Claims 1 to 10, **characterized in that** the functionalized silicone is present in a proportion of from 0.01% to 20% by weight, preferably from 0.1% to 10% by weight, relative to the total weight of the composition.

12. Composition according to any one of Claims 1 to 11, **characterized in that** the detergent surfactant is chosen from anionic, amphoteric, nonionic and cationic surfactants, and mixtures thereof.

13. Composition according to Claim 12, **characterized in that** the anionic surfactants are chosen from alkaline salts, magnesium salts, ammonium salts, amine salts amino alcohol salts of the following compounds: alkyl sulphates, alkyl ether sulphates, alkylamido ether sulphates, alkylaryl polyether sulphates, monoglyceride sulphates; alkyl sulphonates, alkylamide sulphonates, alkylaryl sulphonates, olefin sulphonates, paraffin sulphonates; alkyl sulphosuccinates, alkyl ether sulphosuccinates, alkylamide sulphosuccinates; alkyl sulphosuccinamates; alkyl sulphoacetates; alkyl phosphates, alkyl ether phosphates; acyl sarcosinates, acyl isethionates and N-acyl taurates; the alkyl or acyl radical in these various compounds generally consists of a carbon-based chain containing from 8 to 30 carbon atoms; fatty acid salts of oleic, ricinoleic, palmitic and stearic acid; coconut oil acid or hydrogenated coconut oil acid; acyl lactylates, in which the acyl radical contains from 8 to 30 carbon atoms; alkyl D-galactosiduronic acids and their salts, polyoxyalkylenated alkyl or alkylaryl ether carboxylic acids or their salts, and polyoxyalkylenated alkylamido ether carboxylic acids or their salts.

14. Composition according to Claim 12, **characterized in that** the nonionic surfactants are chosen from polyethoxylated, polyoxypropylenated or polyglycerolated fatty acids or alkylphenols or alcohols, with a fatty chain containing 8 to 30 carbon atoms, the number of ethylene oxide or propylene oxide groups being between, 2 and 50 and the number of glycerol groups being between 2 and 30; copolymers of. ethylene oxide and propylene oxide; condensates of ethylene oxide and of propylene oxide with fatty alcohols; polyethoxylated fatty amides; polyglycerolated fatty amides; polyethoxylated fatty amines; oxyethylenated fatty acid esters of sorbitan; fatty acid esters of sucrose, fatty acid esters of polyethylene glycol; alkylpolyglycosides; carbamate or amide derivatives of N-alkylglucamines, aldobionamides and amine oxides.

15. Composition according to Claim 12, **characterized in that** the amphoteric surfactants are chosen from secondary or tertiary aliphatic amine derivatives, in which the aliphatic radical is a linear or branched chain containing 8 to 22 carbon atoms and which contains at least one carboxylate, sulphonate, sulphate, phosphate or phosphonate water-solubilizing anionic group; (C₈-C₂₀)alkylbetaines, sulphobetaines, (C₈-C₂₀)alkyl-amido(C₁-C₆)alkylbetaines or (C₈-C₂₀)alkylamido(C₁-C₆)alkylsulphobetaines.

16. Composition according to Claim 11, **characterized in that** the cationic surfactants are chosen from quaternary ammonium salts and preferably from dilauryldimethylammonium chloride, diisobutylphenoxy-ethoxyethyldimethylbenzylammonium chloride, cetyltrimethylammonium bromide, N-cetylpyridinium.bromide and benzethonium chloride.

17. Composition according to any one of Claims 1 to 16, **characterized in that** the detergent surfactant is present in a proportion of at least 4% by weight, preferably from 5% to 50% by weight and even more preferably from 8% to 35% by weight, relative to the total weight of the composition.

18. Composition according to any one of Claims 1 to 17, **characterized in that** it has a pH of between 3 and 12 and even more particularly between.4 and 8.

19. Composition according to any one of Claims 1 to 18, **characterized in that** the cosmetically acceptable medium consists of water, of one or more solvents or of a mixture of water and at least one solvent chosen from lower alcohols, alkylene glycols and polyol ethers.

20. Composition according to any one of Claims 1 to 19, **characterized in that** it also contains at least one polyorganosiloxane other than the functionalized silicones as defined in Claims 8 to 11, preferably chosen from linear polydimethylsiloxanes containing trimethylsilyl or hydroxydimethylsilyl end groups.

21. Composition according to any one of Claims 1 to 20, **characterized in that** it also contains at least one cationic polymer.

22. Composition according to Claim 21, **characterized in that** the cationic polymer is chosen from cellulose ether derivatives comprising quaternary ammonium groups, polysaccharides and in particular cationic guar gums, and cyclopolymers of methyldiallylamine or of dimethyldiallylammonium.

23. Composition according to Claim 22, **characterized in that** it contains a cationic polymer in proportions of between 0.001% and 20% by weight and preferably between 0.05% and 5% by weight relative to the total weight of the composition.

24. Composition according to any one of Claims 1 to 23, **characterized in that** it also contains at least one cosmetically acceptable adjuvant chosen from fragrances, preserving agents, sequestering agents, wetting agents, sugars, plant, animal, mineral or synthetic oils, amphoteric polymers, menthol, nicotinate derivatives, agents for preventing hair loss, antidandruff agents, foam stabilizers, propellants, screening agents, dyes, ceramides, vitamins or provitamins and acidifying or basifying agents.

25. Use, as a shampoo, of the composition as defined in any one of Claims 1 to 24.

26. Process for washing keratin materials, **characterized in that** at least one composition as defined in any one of Claims 1 to 24 is applied to the wet or dry keratin materials and, after an optional period of leaving to stand on these materials, they are rinsed with water.
